# EUROPÄISCHE PATENTSCHRIFT

(11) **EP 1 465 582 B1**
(45) Veröffentlichungstag und Bekanntmachung des Hinweises auf die Patenterteilung: **13.10.2010**
(21) Anmeldenummer: 03704358.5
(22) Anmeldetag: 07.01.2003
(51) Int. Cl.: A61Q 5/12, A61K 8/60, C07H 15/02

(54) **ALKYL-UND/ODER ALKYLENOLIGOGLYCOSID-BETAINESTERQUATS**
ALKYL- AND/OR ALKYLENE OLIGOGLYCOSIDE BETAINE ESTER QUATERNARIES
ESTERS QUATERNAIRES DE BETAINE ALKYLOLIGOGLUCOSIDE ET/OU D'ALKYLENOLIGOGLUCOSIDE

(30) Priorität: 16.01.2002 DE 10201354
(43) Veröffentlichungstag der Anmeldung: 13.10.2004
(73) Patentinhaber: Cognis IP Management GmbH, 40589 Düsseldorf (DE)
(72) Erfinder: BEHLER, Ansgar, 46240 Bottrop (DE); CLASEN, Frank, 40721 Hilden (DE)
(86) Internationale Anmeldenummer: PCT/EP2003/000062
(87) Internationale Veröffentlichungsnummer: WO 2003/059298

(56) Entgegenhaltungen:
- WO-A-02/07684
- DE-A- 19 917 745

## Beschreibung

### Gebiet der Erfindung

Die Erfindung betrifft Alk(en)yloligoglycosid-Betainesterquats, ein Verfahren zu deren Herstellung sowie deren Verwendung als Emulgator, Haarconditioner und Wäscheweichspüler.

### Stand der Technik

Kationische Tenside, wie beispielsweise Esterquats, gewinnen aufgrund ihrer ausgezeichneten ökotoxikologischen Eigenschaften sowohl für den Bereich der Wäscheweichspülmittel als auch für kosmetische Anwendungen zunehmend an Bedeutung. In kosmetischen Zubereitungen können sie dabei sowohl in Emulsionen und Lotionen zur Hautpflege wie auch in tensidischen Mitteln, wie beispielsweise Shampoos, Duschbädem, Spülungen, Conditionem und dergleichen, für die Haarpflege enthalten sein. Im Markt besteht nach wie vor der Bedarf neue kationische Tenside zur Verfügung zu stellen, die sich aus natürlichen Rohstoffquellen gewinnen lassen und eine hohe Hydrophilie aufweisen im Vergleich zu nur aus Alkylketten aufgebauten Verbindungen (z.B. Cetyltrimethylammoniumchlorid). Dabei sollte diese höhere Hydrophilie nicht durch Einführung von Alkoxidgruppen erzielt werden, um unter anderem eine geringere Umweltbelastung zu erzielen.

Die Aufgabe der Erfindung hat folglich darin bestanden, neue kationische Tenside sowie ein Verfahren zu ihrer Herstellung zur Verfügung zu stellen, die sich aus einer natürlichen Rohstoffquelle herstellen lassen, ethylenoxid- und/oder propylenoxidfrei sind und damit eine geringere Umweltbelastung darstellen.

### Beschreibung der Erfindung

Gegenstand der vorliegenden Erfindung sind Alk(en)yloligoglycosid-Betainesterquats der Formel **(I)**

**R¹O(G)ₙOCOCR²R³NR⁴R⁵R⁶** **(I)**

in der R¹ für einen Alk(en)ylrest mit 4 bis 22 Kohlenstoffatomen, G für einen Zuckerrest mit 5 oder 6 Kohlenstoffatomen und n für Zahlen von 1 bis 10, R² für H oder eine CH₃-Gruppe, R³ für H oder eine lineare und/oder verzweigte Alk(en)ylgruppe mit 1 bis 6 Kohlenstoffatomen, R⁴, R⁵ und R⁶ unabhängig voneinander für eine lineare und/oder verzweigte Alk(en)ylgruppe mit 1 bis 24 Kohlenstoffatomen oder eine lineare und/oder verzweigte Hydroxyalk(en)ylgruppe mit 1 bis 24 Kohlenstoffatomen steht.

Ein weiterer Gegenstand der Erfindung betrifft ein Verfahren zur Herstellung von Alk(en)yloligoglycosid-Betainesterquats, bei dem man Alk(en)yloligoglycoside der Formel **(II),**

**R¹O(G)ₙ** **(II)**

in der R¹ für einen Alk(en)ylrest mit 4 bis 22 Kohlenstoffatomen, G für einen Zuckerrest mit 5 oder 6 Kohlenstoffatomen und n für Zahlen von 1 bis 10 steht, mit einer α-Halogencarbonsäure der Formel **(III),**

**XCR²R³COOH** **(III)**

in der R² für H oder eine CH₃-Gruppe, R³ für H oder eine lineare und/oder verzweigte Alk(en)ylgruppe mit 1 bis 6 Kohlenstoffatomen und X für Halogen steht, und anschließend mit tertiären Aminen der Formel **(IV),**

**NR⁴R⁵R⁶** **(IV)**

in der R⁴, R⁵ und R⁶ unabhängig voneinander für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 24 Kohlenstoffatomen steht, umsetzt.

Überraschenderweise wurden gefunden, dass man Alk(en)yloligoglycosid-Betainesterquats als neue kationische Zuckertenside durch Umsetzung von Alk(en)yloligoglycosiden mit α-Halogencarbonsäure und tertiären Aminen herstellen kann. Besonders vorteilhaft ist, dass sich diese neuen Tenside aus einer natürlichen Rohstoffquelle ableiten, ethylenoxid- und/oder propylenoxidfrei sind und damit eine geringere Umweltbelastung darstellen. Darüber hinaus sollten die neuen kationischen Tenside eine hohe Hydrophilie aufweisen. Darüber eignen sich diese Verbindungen als Conditioner für die Haare (Haarspülung) und für Textilien (Wäscheweichspüler).

### Alk(en)yloligoglycosid-Betainesterquats

Die vorliegende Erfindung betrifft Alk(en)yloligoglycosid-Betainesterquats [Alk(en)yl = Alkyl- und/oder Alkenyl] der Formel **(I)**

**R¹O(G)ₙOCOCR²R³NR⁴R⁵R⁶** **(I)**

in der R¹ für einen Alk(en)ylrest mit 4 bis 22 Kohlenstoffatomen, G für einen Zuckerrest mit 5 oder 6 Kohlenstoffatomen und n für Zahlen von 1 bis 10, R² für H oder eine CH₃-Gruppe, R³ für H oder eine lineare und/oder verzweigte Alk(en)ylgruppe mit 1 bis 6 Kohlenstoffatomen, R⁴, R⁵ und R⁶ unabhängig voneinander für eine lineare und/oder verzweigte Alk(en)ylgruppe mit 1 bis 24 Kohlenstoffatomen oder eine lineare und/oder verzweigte Hydroxyalk(en)ylgruppe mit 1 bis 24 Kohlenstoffatomen steht.
In einer bevorzugten Ausführungsform der Erfindung werden Alk(en)yloligoglycosid-Betainesterquats der Formel **(I)** eingesetzt, in der R¹ für einen Alk(en)ylrest mit 4 bis 22 Kohlenstoffatomen, G für einen Zuckerrest mit 5 oder 6 Kohlenstoffatomen und n für Zahlen von 1 bis 10, R² und R³ für H oder eine CH₃-Gruppe und vorzugsweise H, R⁴ und R⁵ unabhängig voneinander für eine lineare und/oder verzweigte Alk(en)ylgruppe mit 1 bis 6 Kohlenstoffatomen oder eine lineare und/oder verzweigte Hydroxyalkyl- und/oder Hydroxyalkenylgruppe mit 1 bis 6 Kohlenstoffatomen und vorzugsweise für eine CH₃- oder für eine Hydroxyethyl-Gruppe, R⁶ für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 24 Kohlenstoffatomen steht.

In einer weiteren Ausführungsform der Erfindung werden Alk(en)yloligoglycosid-Betainesterquats der Formel **(I)** eingesetzt, in der R¹ für einen Alk(en)ylrest mit 4 bis 22 Kohlenstoffatomen, G für einen Zuckerrest mit 5 oder 6 Kohlenstoffatomen und n für Zahlen von 1 bis 10, R² und R³ für H, R⁴ und R⁵ für eine CH₃- oder für eine Hydroxyethyl-Gruppe, R⁶ für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 24 Kohlenstoffatomen steht.

Die erfindungsgemäßen Alk(en)yloligoglycosid-Betainesterquats werden in oberflächenaktiven Zubereitungen, vorzugsweise in Wasch-, Spül- und Reinigungsmittel sowie kosmetischen und/oder pharmazeutischen Zubereitungen in Mengen von 0,01 bis 60, vorzugsweise 0,05 bis 30 und insbesondere 2,5 bis 20 Gew.-% - bezogen auf den Aktivsubstanzgehalt - eingesetzt.

### Herstellung von Alk(en)ylglycerinethercarbonsäuren

Die erfindungsgemässen Alk(en)yloligoglycosid-Betainesterquats werden durch Umsetzung von Alk(en)yloligoglycosiden der Formel **(II)** mit α-Halogencarbonsäuren der Formel **(III)** und tertiären Aminen der Formel **(IV)** erhalten. Die bevorzugten Alk(en)yloligoglycosid-Betainesterquats, die über diese Umsetzung erhalten werden können, wurden bereits im vorherigen Kapitel aufgeführt.

Alkyl- und Alkenyloligoglykoside stellen bekannte nichtionische Tenside dar, die der Formel **(II)** folgen,

**R¹O-[G]ₙ** **(II)**

in der R¹ für einen Alk(en)ylrest mit 4 bis 22 Kohlenstoffatomen, G für einen Zuckerrest mit 5 oder 6 Kohlenstoffatomen und n für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Staren/Stärke 45, 281 (1993**),** B. Salka in Cosm.Toil.108, 89 (1993**)** sowie J.Kahre et al. in SÖFW-Journal Heft 8, 598 (1995**)** verwiesen.
Die Alk(en)yloligoglykoside können sich von Aldosen bzw. Ketosen mit 5 oder 6 Kohlenstoffatomen, vorzugsweise der Glucose ableiten. Die bevorzugten Alk(en)yloligoglykoside sind somit Alk(en)yloligoglucoside. Die Indexzahl n in der allgemeinen Formel **(II)** gibt den Oligomerisierungsgrad (DP), d. h. die Verteilung von Mono- und Oligoglykosiden an und steht für eine Zahl zwischen 1 und 10. Während n in einer gegebenen Verbindung stets ganzzahlig sein muss und hier vor allem die Werte n = 1 bis 6 annehmen kann, ist der Wert n für ein bestimmte Alkyloligoglykosid eine analytisch ermittelte rechnerische Größe, die meistens eine gebrochene Zahl darstellt. Vorzugsweise werden Alk(en)yloligoglykoside mit einem mittleren Oligomerisierungsgrad n von 1,1 bis 3,0 eingesetzt. Aus anwendungstechnischer Sicht sind solche Alk(en)yloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich vorzugsweise von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelenschen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alk(en)ylrest R¹ kann sich ferner vorzugsweise von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Besonders bevorzugt sind Alkyloligoglucoside auf Basis von gehärtetem C_{12/14}-Kokosalkohol mit einem DP von 1 bis 3.

α-Halogencarbonsäuren sind nach den einschlägigen Verfahren der organischen Chemie erhältlich und folgen der Formel **(III)**,

**XCR²R³COOH** **(III)**

in der R² für H oder eine CH₃-Gruppe, R³ für H oder eine lineare und/oder verzweigte Alk(en)ylgruppe mit 1 bis 6 Kohlenstoffatomen und X für Halogen steht. In einer besonderen Ausführungsform der Erfindung stehen R² und R³ für H oder eine CH₃-Gruppe und insbesondere für H. Insbesondere wird α-Halogenessigsäure, wie beispielsweise Monochloressigsäure eingesetzt.

Tertiären Amine, die im Sinne der Erfindung eingesetzt werden können, folgen der Formel **(IV),**

**NR4R5R6** **(IV)**

in der R⁴, R⁵ und R⁶ unabhängig voneinander für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 24 Kohlenstoffatomen steht. In einer besonderen Ausführungsform der Erfindung stehen R⁴ und R⁵ unabhängig voneinander für eine lineare und/oder verzweigte Alk(en)ylgruppe mit 1 bis 6 Kohlenstoffatomen oder eine lineare und/oder verzweigte Hydroxyalkyl- und/oder Hydroxyalkenylgruppe mit 1 bis 6 Kohlenstoffatomen und R⁶ für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 24 Kohlenstoffatomen.
In einer weiteren Ausführungsform der Erfindung stehen R⁴ und R⁵ unabhängig voneinander für eine CH₃- oder eine Hydroxyethylgruppe und R⁶ für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 24 und vorzugsweise für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 6 Kohlenstoffatomen.
Dementsprechend kann sich das tertiäre Amin von kurzkettigen tertiären Aminen, vorzugsweise Dimethylethanolamin, Triethanolamin und Methyldiethanolamin oder langkettigen Aminen, vorzugsweise Dimethylkokosamin, Dimethyllauryamin und Dimethyltalgamin ableiten.

Zur Herstellung der erfindungsgemässen Verbindungen wird das Alk(en)yloligoglycosid zuvor bis zu einem Wassergehalt von maximal 5, vorzugsweise maximal 4 Gew.-% - bezogen auf den Aktivsubstanzgehalt des Alk(en)yloligoglycosids - getrocknet und anschliessend zur Umsetzung in einem Reaktionsbehälter vorgelegt. Im Anschluss wird das Alk(en)yloligoglycosid mit der α-Halogencarbonsäure im Molverhältnis 1 : 0,5 bis 1 : 3 und vorzugsweise 1 : 1 bis 1 : 1,5 unter Zusatz von bis zu 50 % eines organischen Lösungsmittels bezogen auf den Gesamtansatz bei einer Temperaturen von 100 bis 130 und vorzugsweise 115 bis 120 °C unter Wasserabscheidung für 5 bis 13 h und vorzugsweise 7 bis 12 h verestert. Vorzugsweise werden als organisches Lösungsmittel Toluol, Benzol und Xylol und insbesondere Toluol eingesetzt. Im Anschluss daran wird das erhaltene Reaktionsprodukt mit einem tertiären Amin der Formel **(IV)** im Molverhältnis 1 : 0,5 bis 1 : 3 und vorzugsweise 1 : 1 bis 1 : 1,5 bei einer Temperatur von 70 bis 100 und vorzugsweise 80 bis 90 °C umgesetzt. Hierbei wird das tertiäre Amin zuvor mit soviel organischem Lösungsmittels versetzt, dass eine gut rührfähige Reaktionsmischung vorliegt. Die Reaktion war mit Erreichen der theoretischen Menge an freiwerdendem anorganischen Halogenid abgeschlossen. Üblicherweise betragen die Reaktionszeiten 30 Minuten bis 3 Stunden und vorzugsweise 50 Minuten bis 1,5 Stunden.

### Gewerbliche Anwendbarkeit

Die erfindungsgemässen Alk(en)yloligoglycosid-Betainesterquats können durch Zusatz von Wasser auf beliebige Konzentrationen eingestellt werden, wobei der Wassergehalt vorzugsweise 20 bis 85, besonders bevorzugt 20 bis 75 und insbesondere 50 bis 70 Gew.-% betragen kann.

Die erfindungsgemässen Alk(en)yloligoglycosid-Betainesterquats können als Tenside in oberflächenaktiven Zubereitungen verwendet werden. Unter oberflächenaktiven Zubereitungen werden im Sinne der Erfindung vorzugsweise Wasch- und Spül- und Reinigungsmittel sowie kosmetische und/oder pharmazeutische Zubereitungen und insbesondere kosmetische und/oder pharmazeutische Zubereitungen verstanden. Insbesondere können die erfindungsgemäßen Produkte in Haarconditionem und Wäscheweichspülmitteln eingesetzt werden. Diese oberflächenaktiven Zubereitungen können als weitere Hilfs-und Zusatzstoffe Perlglanzwachse, Konsistenzgeber, Verdickungsmittel, Überfettungsmittel, Stabilisatoren, Siliconverbindungen, Fette, Wachse, Antioxidantien, Antischuppenmittel, Quellmittel, Tyroininhibitoren, Hydrotrope, Solubilisatoren, Konservierungsmittel, Parfümöle, Farbstoffe, weitere Tenside sowie weitere typische Inhaltsstoffe, wie sie beispielsweise in Wasch- Spül und Reinigungsmitteln vorkommen, enthalten. Als kosmetische und/oder pharmazeutische Zubereitungen kommen vorzugsweise Mund- und Zahnpflegemittel, Haarshampoos, Haarlotionen, Schaumbäder, Duschbäder, Cremes, Lotionen, Gele, Emulsionen, Wachs/Fett-Massen, Stiftpräparaten oder Salben in Frage. Diese oberflächenaktiven Zubereitungen können neben den erfindungsgemässen Alk(en)yloligoglycosid-Betainesterquats weitere aus dem Stand der Technik für die jeweilige Anwendung typischen Inhaltsstoffe in üblichen Konzentrationen enthalten.

Die erfindungsgemässen Mittel zeigen nicht nur conditionierende [Conditioner für Haare (Haarspülung) und Textilien (Wäscheweichspüler)] sondern darüber hinaus ebenfalls schäumende und reinigende Eigenschaften. Darüber hinaus können höherkettige Alk(en)yloligoglycosid-Betainesterquats wie beispielsweise C_{16/18}-Oligoglycosid-Betainesterquats emulgierende Eigenschaften aufweisen und somit in kosmetischen und/oder pharmazeutischen Zubereitungen eingesetzt werden. Weitere Gegenstände der Erfindung sind somit auf die Verwendung der erfindungsgemässen Alk(en)yloligoglycosid-Betainester quats als Conditioner für die Haare, als Weichspüler und als Emulgator gerichtet. Insbesondere können C_{16/18}-Alkyloligoglycosid-Betainesterquats als Emulgatoren in allen dem Fachmann bekannten Emulsions-Typen verwendet.

Typische kosmetische und/oder pharmazeutische Reinigungsmittel weisen vorzugsweise folgende Zusammensetzung auf - bezogen auf den Aktivsubstanzgehalt - :
(a) 0,05 bis 20, vorzugsweise 0,5 bis 10 und insbesondere 2,5 bis 18 Gew.-% Alk(en)yloligoglycosid-Betainesterquats,
(b) 0,05 bis 15, vorzugsweise 0,5 bis 10 und insbesondere 2,5 bis 7,5 Gew.-% Betaine und gegebenenfalls
(c) 0 bis 15, vorzugsweise 0,5 bis 10 und insbesondere 2,5 bis 7,5 Gew.-% Aniontenside mit der Massgabe, dass sich die Mengenangaben mit Wasser und gegebenenfalls weiteren Hilfs- und Zusatzstoffen zu 100 Gew.-% ergänzen.

Typische flüssige Wasch- und Spülmittel, Reinigungsmittel weisen vorzugsweise folgende Zusammensetzung auf - bezogen auf den Aktivsubstanzgehalt - :
(a) 2,5 bis 30, vorzugsweise 7 bis 25 und insbesondere 10 bis 20 Gew.-% Alk(en)yloligoglycosid-Betainesterquats,
(b) 0,05 bis 15, vorzugsweise 0,5 bis 10 und insbesondere 2,5 bis 7,5 Gew.-% Betaine und gegebenenfalls
(c) 2,5 bis 30, vorzugsweise 7 bis 25 und insbesondere 10 bis 20 Gew.-% Aniontenside mit der Maßgabe, dass sich die Mengenangaben mit Wasser und gegebenenfalls weiteren Hilfs- und Zusatzstoffen zu 100 Gew.-% ergänzen.

Typische kosmetische und/oder pharmazeutische Emulsionen, weisen vorzugsweise folgende Zusammensetzung auf- bezogen auf den Aktivsubstanzgehalt :
(a) 0,05 bis 15, vorzugsweise 0,5 bis 10 und insbesondere 1 bis 5 Gew.-% Alk(en)yloligoglycosid-Betainesterquats und vorzugsweise C_{16/18}-Alkyloligoglycosid-Betainesterquats,
(b) 3 bis 30, vorzugsweise 5 bis 20 und insbesondere 7 bis 15 Gew.-% Ölkörper und gegebenenfalls
(c) 0,5 bis 20 und vorzugsweise 2,5 bis 10 Gew.-% Gew.-% Konsistentgeber mit der Massgabe, dass sich die Mengenangaben mit Wasser und gegebenenfalls weiteren Hilfs- und Zusatzstoffen zu 100 Gew.-% ergänzen.

### Beispiele

Die folgenden Beispiele sollen den Gegenstand der Erfindung näher erläutern, ohne ihn hierauf zu beschränken.

### Beispiel 1:

### (a) Herstellung von C_{12/14}-Alkyloligoglycosid-Chloressigsäureester

In einem 11-Dreihalskolben wurden 214,0 (0,5 mol) eines mit Citronensäure neutralisierten und gefriergetrockneten (wasserfreien, 1,7 Gew.-% Wasser) C_{12/14}- Alkyloligoglycosids (Basis Plantacare 1200 UP, Fa. Cognis) mit 61,4 g (0,65 mol) Chloressigsäure unter Zusatz von 250 ml Toluol bei 115-120 °C unter Wasserabscheidung versetzt. Die Reaktion war nach 11,25 h beendet. Man erhielt 513,4 g eines dunkelgelben, trüben und flüssigen Produktes. Der Umsatz an Alkyloligoglucosid betrug 70,6 %.

| | |
|---|---|
| Säurezahl : | 19,9 |
| Verseifungszahl: | 158,0 |
| Freie Monoglucoside (GC) | 8,1 % |
| Freie Diglucoside (GC) | 4,6 % |
| Freie Triglucoside (GC) | 0,8 % |
| Freie Tetraglucoside (GC) | 0,3 % |
| Freie Pentaglucoside (GC) | 0,1 % |

### (b) Herstellung des kationischen C_{12/14}-Alkyloligoglycosid-Betainesterquat

In einem 500-ml-Dreihalskolben wurden 29,0 (0,3 mol) des in (a) hergestellten C_{12/14}-Alkyloligoglycosid-Chloressigsäureesters mit 27,6 g (0,3 mol) N,N-Dimethylethanolamin in 220 g Toluol bei 80 °C umgesetzt. Die Reaktion war nach Erreichen der theoretischen Menge an freiwerdendem anorganischen Chlorid (2,82 %) nach 1,25 h abgeschlossen. Am Rotavapor wurde bei 60 bis 80 °C unter Vakuum (35 mbar) das Lösungsmittel Toluol abdestilliert. Das Produkt wurde mit Wasser auf eine Konzentration von 30 % Aktivsubstanz eingestellt. Es lag als dunkelbraunes, flüssiges Produkt vor.

### (c) Herstellung eines kationischen C12/14-Alkyloligoglucosid-Betainesterquat auf Basis N,N-Dimethyl-Octyl/decylamin

In einem 250 ml-Dreihalskolben wurden 41,5 g (96,7 mmol) des in a) hergestellten C_{12/14}-APG-Chloressigsäureesters mit 17,2 g (96,7 mmol) N,N-Dimethyl-Octyl/Decylamin in 36,7 g Toluol bei 80°C umgesetzt.

Die Reaktion war nach Erreichen der theoretischen Menge an freiwerdendem anorganischen Chlorid (4,05%) nach 1h10min abgeschlossen. Nach Zugabe von 250 g Wasser wurde das Toluol an einem Wasserabscheider für schwere Lösungsmittel abdestilliert. Der pH-Wert wurde anschließend auf 6,4 eingestellt. Es lag ein gelbes, homogenes, flüssiges Produkt vor.

| | |
|---|---|
| Kationentensidgehalt: | 17,4% |
| Trockenrückstand: | 24,0 % |

## Patentansprüche

1. Alk(en)yloligoglycosid-Betainesterquats der Formel **(I)**
**R¹O(G)ₙOCOCR²R³NR⁴R⁵R⁶** (I)
in der R¹ für einen Alk(en)ylrest mit 4 bis 22 Kohlenstoffatomen, G für einen Zuckerrest mit 5 oder 6 Kohlenstoffatomen und n für Zahlen von 1 bis 10, R² für H oder eine CH₃-Gruppe, R³ für H oder eine lineare und/oder verzweigte Alk(en)ylgruppe mit 1 bis 6 Kohlenstoffatomen, R⁴, R⁵ und R⁶ unabhängig voneinander für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 24 Kohlenstoffatomen steht.

2. Verbindungen der Formel (I) nach Anspruch 1, **dadurch gekennzeichnet, dass** R¹ für einen Alk(en)ylrest mit 4 bis 22 Kohlenstoffatomen, G für einen Zuckerrest mit 5 oder 6 Kohlenstoffatomen und n für Zahlen von 1 bis 10, R² und R³ für H oder eine CH₃-Gruppe, R⁴ und R⁵ unabhängig voneinander für eine lineare und/oder verzweigte Alk(en)ylgruppe mit 1 bis 6 Kohlenstoffatomen oder eine lineare und/oder verzweigte Hydroxyalkyl- und/oder Hydroxyalkenylgruppe mit 1 bis 6 Kohlenstoffatomen, R⁶ für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 24 Kohlenstoffatomen steht.

3. Verbindungen der Formel (I) nach einem der Ansprüche 1 und/oder 2, **dadurch gekennzeichnet, dass** R¹ für einen Alk(en)ylrest mit 4 bis 22 Kohlenstoffatomen, G für einen Zuckerrest mit 5 oder 6 Kohlenstoffatomen und n für Zahlen von 1 bis 10, R² und R³ für H, R⁴ und R⁵ für eine CH₃- oder für eine Hydroxyethyl- Gruppe, R⁶ für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 24 Kohlenstoffatomen steht.

4. Oberflächenaktive Zubereitungen **dadurch gekennzeichnet, dass** sie Alk(en)yloligoglycosid-Betainesterquats nach mindestens einem der Ansprüche 1 bis 3 enthalten.

5. Wasch-, Spül- und Reinigungsmittel sowie kosmetische und/oder pharmazeutische Zubereitungen **dadurch gekennzeichnet, dass** sie Alk(en)yloligoglycosid-Betainesterquats nach mindestens einem der Ansprüche 1 bis 3 in Mengen von 0,01 bis 60 Gew.-% - bezogen auf den Aktivsubstanzgehalt - enthalten.

6. Verfahren zur Herstellung von Alk(en)yloligoglycosid-Betainesterquats, der Formel (I) nach Anspruch 1, bei dem man Alk(en)yloligoglycoside der Formel **(II),**
**R¹O(G)ₙ** **(II)**
in der R¹ für einen Alk(en)ylrest mit 4 bis 22 Kohlenstoffatomen, G für einen Zuckerrest mit 5 oder 6 Kohlenstoffatomen und n für Zahlen von 1 bis 10 steht, mit einer α-Halogencarbonsäure der Formel **(III),**
**XCR²R³COOH** **(III)**
in der R² für H oder eine CH₃-Gruppe, R³ für H oder eine lineare und/oder verzweigte Alk(en)ylgruppe mit 1 bis 6 Kohlenstoffatomen und X für Halogen steht, und anschließend mit einem tertiären Aminen der Formel **(IV),**
**NR⁴R⁵R⁶** **(IV)**
in der R⁴, R⁵ und R⁶ unabhängig voneinander für eine lineare und/oder verzweigte Alk(en)ylgruppe mit 1 bis 24 Kohlenstoffatomen oder eine lineare und/oder verzweigte Hydroxyalkyl- und/oder Hydroxyalkenylgruppe mit 1 bis 24 Kohlenstoffatomen steht, umsetzt.

7. Verwendung der Alk(en)yloligoglycosid-Betainesterquats, der Formel (I) nach Anspruch 1 als Emulgator.

8. Verwendung der Alk(en)yloligoglycosid-Betainesterquats der Formel (I) nach Anspruch 1 als Conditioner für die Haare.

9. Verwendung der Alk(en)yloligoglycosid-Betainesterquats der Formel (I) nach Anspruch 1 als Wäscheweichspüler.

## Claims

1. Alk(en)yl oligoglycoside betaine esterquats corresponding to formula **(I):**
**R¹O(G)ₙOCOCR²R³NR⁴R⁵R⁶** **(I)**
in which R¹ is an alk(en)yl group containing 4 to 22 carbon atoms, G is a sugar unit containing 5 or 6 carbon atoms and n is a number of 1 to 10, R² is H or a CH₃ group, R³ is H or a linear and/or branched alk(en)yl group containing 1 to 6 carbon atoms, R⁴, R⁵ and R⁶ independently of one another represent a linear and/or branched alk(en)yl group containing 1 to 24 carbon atoms or a linear and/or branched hydroxyalkyl and/or hydroxyalkenyl group containing 1 to 24 carbon atoms.

2. Compounds according to formula **(I)** as claimed in claim 1 in which R¹ is an alk(en)yl group containing 4 to 22 carbon atoms, G is a sugar unit containing 5 or 6 carbon atoms and n is a number of 1 to 10, R² and R³ are H or a CH₃ group, R⁴ and R⁵ independently of one another represent a linear and/or branched alk(en)yl group containing 1 to 6 carbon atoms or a linear and/or branched hydroxyalkyl and/or hydroxyalkenyl group containing 1 to 6 carbon atoms, R⁶ is a linear and/or branched alk(en)yl group containing 1 to 24 carbon atoms or a linear and/or branched hydroxyalkyl and/or hydroxyalkenyl group containing 1 to 24 carbon atoms.

3. Compounds according to formula **(I)** as claimed in claim(s) 1 and/or 2 in which R¹ is an alk(en)yl group containing 4 to 22 carbon atoms, G is a sugar unit containing 5 or 6 carbon atoms and n is a number of 1 to 10, R² and R³ are H, R⁴ and R⁵ represent a CH₃ group or a hydroxyethyl group, R⁶ is a linear and/or branched alk(en)yl group containing 1 to 24 carbon atoms or a linear and/or branched hydroxyalkyl and/or hydroxyalkenyl group containing 1 to 24 carbon atoms.

4. Surface-active preparations **characterized in that** they contain alk(en)yl oligoglycoside betaine esterquats according to at least one of the claims 1 to 3.

5. Laundry detergents, dishwashing detergents and cleaners and cosmetic and/or pharmaceutical preparations, **characterized in that** they contain alk(en)yl oligoglycoside betaine esterquats according to at least one of the claims 1 to 3 in quantities of 0.01 to 60% by weight, based on the active substance content.

6. A process for the production of alk(en)yl oligoglycoside betaine esterquats of formula **(I)** of claim 1, in which alk(en)yl oligoglycosides corresponding to formula **(II):**
**R¹O(G)ₙ** **(II)**
in which R¹ is an alk(en)yl group containing 4 to 22 carbon atoms, G is a sugar unit containing 5 or 6 carbon atoms and n is a number of 1 to 10, are reacted with an α-halocarboxylic acid corresponding to formula **(III):**
**XCR²R³COOH** **(III)**
in which R² is H or a CH₃ group, R³ is H or a linear and/or branched alk(en)yl group containing 1 to 6 carbon atoms and X is halogen, and then with a tertiary amine corresponding to formula **(IV):**
**NR⁴R⁵R⁶** **(IV)**
in which R⁴, R⁵ and R⁶ independently of one another represent a linear and/or branched alk(en)yl group containing 1 to 24 carbon atoms or a linear and/or branched hydroxyalkyl and/or hydroxyalkenyl group containing 1 to 24 carbon atoms.

7. The use of alk(en)yl oligoglycoside betaine esterquats of formula **(I)** in claim 1 as an emulsifier.

8. The use of alk(en)yl oligoglycoside betaine esterquats of formula **(I)** of claim 1 as a hair conditioner.

9. The use of alk(en)yl oligoglycoside betaine esterquats of formula **(I)** of claim 1 as a fabric softener.

## Revendications

1. Esters quaternaires d'alkyloligoglycoside-bétaïne ou d'alcényloligoglycoside-bétaïne de formule (I)
R¹O(G)ₙOCOCR²R³NR⁴R⁵R⁶ (I)
dans laquelle R¹ représente un radical alkyle ou alcényle comprenant 4 à 22 atomes de carbone, G représente un radical de sucre comprenant 5 ou 6 atomes de carbone et n représente des nombres de 1 à 10, R² représente H ou un groupe CH₃, R³ représente H ou un groupe alkyle ou alcényle linéaire et/ou ramifié comprenant 1 à 6 atomes de carbone, R⁴, R⁵ et R⁶ représentent, indépendamment l'un de l'autre, un groupe alkyle ou alcényle linéaire et/ou ramifié comprenant 1 à 24 atomes de carbone ou un groupe hydroxyalkyle et/ou hydroxyalcényle linéaire et/ou ramifié comprenant 1 à 24 atomes de carbone.

2. Composés de formule (I) selon la revendication 1, **caractérisés en ce que** R¹ représente un radical alkyle ou alcényle comprenant 4 à 22 atomes de carbone, G représente un radical de sucre comprenant 5 ou 6 atomes de carbone et n représente des nombres de 1 à 10, R² et R³ représentent H ou un groupe CH₃, R⁴ et R⁵ représentent, indépendamment l'un de l'autre, un groupe alkyle ou alcényle linéaire et/ou ramifié comprenant 1 à 6 atomes de carbone ou un groupe hydroxyalkyle et/ou hydroxyalcényle linéaire et/ou ramifié, comprenant 1 à 6 atomes de carbone, R⁶ représente un groupe alkyle ou alcényle linéaire et/ou ramifié comprenant 1 à 24 atomes de carbone ou un groupe hydroxyalkyle et/ou hydroxyalcényle linéaire et/ou ramifié comprenant 1 à 24 atomes de carbone.

3. Composés de formule (I) selon l'une quelconque des revendications 1 et/ou 2, **caractérisés en ce que** R¹ représente un radical alkyle ou alcényle comprenant 4 à 22 atomes de carbone, G représente un radical de sucre comprenant 5 ou 6 atomes de carbone et n représente des nombres de 1 à 10, R² et R³ représentent H, R⁴ et R⁵ représentent un groupe CH₃ ou hydroxyéthyle, R⁶ représente un groupe alkyle ou alcényle linéaire et/ou ramifié comprenant 1 à 24 atomes de carbone ou un groupe hydroxyalkyle et/ou hydroxyalcényle linéaire et/ou ramifié comprenant 1 à 24 atomes de carbone.

4. Compositions tensioactives **caractérisées en ce qu'**elles contiennent des esters quaternaires d'alkyloligoglycoside-bétaïne ou d'alcényloligoglycoside-bétaïne selon au moins l'une quelconque des revendications 1 à 3.

5. Agents de lavage, de rinçage et de nettoyage ainsi que compositions cosmétiques et/ou pharmaceutiques, **caractérisés en ce qu'**ils contiennent des esters quaternaires d'alkyloligoglycoside-bétaïne ou d'alcényloligoglycoside-bétaïne selon au moins l'une quelconque des revendications 1 à 3 en des quantités de 0,01 à 60% en poids par rapport à la teneur en substance active.

6. Procédé pour la préparation d'esters quaternaires d'alkyloligoglycoside-bétaïne ou d'alcényloligoglycoside-bétaïne de formule (I) selon la revendication 1, dans lequel on transforme des alkyloligoglycosides ou des alcényloligoglycosides de formule (II),
R¹O(G)ₙ (II)
dans laquelle R¹ représente un radical alkyle ou alcényle comprenant 4 à 22 atomes de carbone, G représente un radical de sucre comprenant 5 ou 6 atomes de carbone et n représente des nombres de 1 à 10, avec un acide α-halogénocarboxylique de formule (III),
XCR²R³COOH (III)
dans laquelle R² représente H ou un groupe CH₃, R³ représente H ou un groupe alkyle ou alcényle linéaire et/ou ramifié comprenant 1 à 6 atomes de carbone et X représente halogène, puis avec une amine tertiaire de formule (IV),
NR⁴R⁵R⁶ (IV)
dans laquelle R⁴, R⁵ et R⁶ représentent, indépendamment l'un de l'autre, un groupe alkyle ou alcényle linéaire et/ou ramifié comprenant 1 à 24 atomes de carbone ou un groupe hydroxyalkyle et/ou hydroxyalcényle linéaire et/ou ramifié comprenant 1 à 24 atomes de carbone.

7. Utilisation des esters quaternaires d'alkyloligoglycoside-bétaïne ou d'alcényloligoglycoside-bétaïne de formule (I) selon la revendication 1 comme émulsifiant.

8. Utilisation des esters quaternaires d'alkyloligoglycoside-bétaïne ou d'alcényloligoglycoside-bétaïne de formule (I) selon la revendication 1 comme agent de conditionnement pour les cheveux.

9. Utilisation des esters quaternaires d'alkyloligoglycoside-bétaïne ou d'alcényloligoglycoside-bétaïne de formule (I) selon la revendication 1 comme adoucissant du linge.
